# EUROPÄISCHE PATENTSCHRIFT

(11) **EP 0 784 643 B1**
(45) Veröffentlichungstag und Bekanntmachung des Hinweises auf die Patenterteilung: **17.06.1998**
(21) Anmeldenummer: 95936452.2
(22) Anmeldetag: 06.10.1995
(51) Int. Cl.: C08G 65/30, C08G 63/90, C07C 41/34

(54) **VERFAHREN ZUR ENTFERNUNG VON HETEROPOLYVERBINDUNGEN AUS POLYETHERN, POLYESTERN UND POLYETHERESTERN**
PROCESS FOR EXTRACTING HETEROPOLYCOMPOUNDS FROM POLYETHERS, POLYESTERS AND POLYETHER ESTERS
PROCEDE D'EXTRACTION DES HETEROPOLYCOMPOSES CONTENUS DANS DES POLYETHERS, POLYESTERS ET ESTERS DE POLYETHER

(30) Priorität: 07.10.1994 DE 4435934
(43) Veröffentlichungstag der Anmeldung: 23.07.1997
(73) Patentinhaber: BASF AKTIENGESELLSCHAFT, 67056 Ludwigshafen (DE)
(72) Erfinder: WEYER, Hans-Jürgen, D-67240 Bobenheim-Roxheim (DE); FISCHER, Rolf, D-69121 Heidelberg (DE)
(74) Vertreter: Isenbruck, Günter, Dr.
(86) Internationale Anmeldenummer: EP9503956
(87) Internationale Veröffentlichungsnummer: WO9611222

(56) Entgegenhaltungen:
- EP-A- 0 000 944
- EP-A- 0 181 621
- EP-A- 0 503 393
- GB-A- 1 369 304

## Beschreibung

Die Erfindung betrifft ein Verfahren zur Entfernung von Heteropolyverbindungen aus derartige Verbindungen enthaltenden Polyethern, Polyestern und/oder Polyetherestern oder deren Lösungen durch Mischen derselben mit einem Ether, der von so geringer Polarität ist, daß seine Zugabe zur Abscheidung der in der Polymerphase vorhandenen Heteropolyverbindung in einer gesonderten Phase führt.

Polyether, Polyester und Polyetherester werden vielseitig verwendet, beispielhaft sei hier deren Verwendung in Hydraulikölen oder als Diolkomponente bei der Herstellung von Polyurethanen genannt. Diese Verbindungen werden durch kationische Polymerisation oder Copolymerisation entsprechender Monomere, wie cyclischer Ether, Acetale, Polyalkohole oder Lactone, mit Hilfe von Brönstedt- oder Lewis-Säure-Katalysatoren hergestellt. Als besonders vorteilhafte Katalysatoren für eine Ringöffnungspolymerisation haben sich Heteropolysäuren und Heteropolysäuresalze, im folgenden gemeinsam als "Heteropolyverbindung(en)" oder "HPA" bezeichnet, erwiesen. Zur Einstellung der gewünschten Polymermolmasse und/oder zur Herstellung spezieller, endgruppenmodifizierter Derivate sind bei der Polymerisation üblicherweise Substanzen anwesend, deren Einbau direkt oder indirekt zum Kettenabbruch führt. Dies sind z.B. Carbonsäurederivate, Alkohole oder Wasser.

JP-A-83 028/1983 beschreibt beispielsweise die Polymerisation von Tetrahydrofuran (THF) in Gegenwart eines Carbonsäureanhydrids oder -halogenids unter Bildung von PolyTHF-Diestern, wobei einer Heteropolysäure als Katalysator benutzt wird.

EP-A-0 126 471 lehrt die HPA-katalysierte Polymerisation von THF und die Copolymerisation von THF mit verschiedenen anderen cyclischen Ethern in Gegenwart von Wasser unter Bildung von Polyetherglykolen. In EP-A-0 158 229 wird die Herstellung von Polyetherglykolen durch Copolymerisation cyclischer Ether mit di- und höherfunktionellen Alkoholen beschrieben.

Nach JP-A-61-200120 können Lactone oder Lactone zusammen mit cyclischen Ethern in Gegenwart von Hydroxylgruppen-haltigen Verbindungen mit Heteropolysäuren als Katalysatoren polymerisiert werden.

Polyetherglykolmonoether und Polyetherglykolmonoester lassen sich nach EP-A-0 503 393 und EP-A-0 503 394 durch Polymerisation cyclischer Ether in Gegenwart von Monoalkoholen oder Monocarbonsäuren mit HPA-Katalysatoren herstellen.

Bei diesen Polymerisationsverfahren zur Herstellung von Polyethern, Polyestern und Polyetherestern entstehen Polymerphasen, die aufgrund des unvollständigen Umsatzes noch verbleibende Mengen an Monomer(en), verbleibende Mengen der zum Kettenabbruch führenden Verbindung(en), gegebenenfalls eingesetzte Lösungsmittel sowie gelöste Heteropolyverbindungen(en) enthalten. Der Prozentsatz an in der Polymerphase gelöstem HPA-Katalysator ist bei diesen Reaktionen beträchtlich und kann bis zu 1 Gew.-% und mehr, bezogen auf die Polymerphase, betragen. Der gelöste Katalysator fällt bei einer Abtrennung, die lediglich aus einem Abdestillieren von unumgesetztem Monomer, Kettenabbruchsreagenz(ien) und ggf. eingesetztem Lösungsmittel aus dieser Phase besteht, nicht aus, sondern verbleibt in gelöster Form im Polymer. Einerseits muß dies aus Qualitätsgründen verhindert werden, andererseits ist es aus Kostengründen, da Heteropolysäuren sehr teuer sind, wünschenswert, den größten Teil des Katalysators wiederzugewinnen.

Weiter ist bekannt, daß sich Heteropolyverbindungen im Lauf der Zeit und verstärkt bei thermischer Belastung zersetzen. Dies geschieht vor allem durch Hydrolyse unter Bildung der entsprechenden Oxide. Die Zersetzung von Heteropolyverbindungen kann u. a. durch die Zugabe eines Ethers verlangsamt oder sogar ganz verhindert werden, wie dies beispielsweise von A. Aoshima, S. Yamamatsu und T. Yamaguchi in Nippon Kagaku Kaishi (1990) 233 beschrieben wurde.

Zur Lösung des Problems der Abtrennung von Heteropolyverbindungen wird in EP-A-0 181 621 vorgeschlagen, die Polymerphase mit einem Kohlenwasserstoff oder halogenierten Kohlenwasserstoff zu versetzen, wodurch der größte Teil der gelösten Heteropolysäure ausfällt und/oder als gesonderte Phase abgeschieden wird. Die abgetrennte Kohlenwasserstoff/Polymer-Phase wird anschließend mit einem festen Adsorbens behandelt. Eine Stabilisierung der Heteropolyverbindungen wird durch das Ausfällen mit einem (ggf. halogenierten) Kohlenwasserstoff in diesem Verfahren jedoch nicht erreicht.

In SU-A1-1754732 wird nach Konzentration der Polymerphase auf 50 -90% Polymer ebenfalls ein Kohlenwasserstoff mit 1 - 15 C-Atomen zur Ausfällung der Heteropolyverbindung zugesetzt. Anstelle einer weiteren Reinigung durch Adsorption wird dann jedoch eine flüssige organische Stickstoffbase zugesetzt, die mit noch vorhandener Heteropolyverbindung, bei der es sich in diesem Fall um die Säure handeln muß, ein unlösliches Salz bildet, das ausfällt und auf übliche Weise abgetrennt werden kann. Die somit zu erzielende Reinheit von ca. 50 x 10⁻⁶% Heteropolyverbindung im Polymer ist jedoch für die meisten Zwecke nicht ausreichend.

Aufgabe der vorliegenden Erfindung war es demgemäß, ein Verfahren zur Reinigung von Heteropolyverbindungen enthaltenden Polyethern, Polyestern und/oder Polyetherestern oder deren Lösungen zu finden, das die Heteropolyverbindungen abtrennen und gleichzeitig zur Stabilisierung der eingesetzten HPA dienen kann.

Diese Aufgabe wird durch ein Verfahren zur Entfernung von Heteropolyverbindungen aus mit Heteropolyverbindungen verunreinigten Polyethern, Polyestern und/oder Polyetherestern, gelöst, das dadurch gekennzeichnet ist, daß diese Polymere oder eine Lösung derselben mit einem Ether versetzt werden, der von so geringer Polarität ist, daß seine Zugabe zur Abscheidung der Heteropolyverbindung aus der Polymerphase in einer gesonderten Phase führt.

Nach dem erfindungsgemäßen Verfahren können Polyether, Polyester und/oder Polyetherester von Heteropolyverbindung(en) gereinigt werden. Dabei kann das beschriebene Reinigungsverfahren für jedes Gemisch, das HPA und eines oder mehrere der genannten Polymere enthält, eingesetzt werden.

Die genannten polymeren Verbindungen können beispielsweise aus Monomeren der Gruppe der cyclischen Ether, Acetale, Diole, höheren Alkohole und Lactone aufgebaut sein. Es ist aber auch möglich, aus anderen Monomeren bestehende polymere Verbindungen nach dem erfindungsgemäßen Verfahren von gelösten Heteropolyverbindungen zu befreien. Die Molmasse der zu reinigenden Polyether, Polyester und/oder Polyetherester ist nicht beschränkt, liegt aber bevorzugt unter 5000.

Man setzt die Heteropolyverbindung(en) enthaltenden Polyether, Polyester und/oder Polyetherester als solche oder in gelöster Form, beispielsweise und bevorzugt in ihrer bei der Polymerisation anfallenden Lösung, ein. In dieser Beschreibung und den Ansprüchen wird als "Polymerphase" oder "(Polymer)gemisch" eine Phase bezeichnet, die mindestens eines der obigen Polymere enthält, während als "Polymerlösung" eine Phase bezeichnet wird, die noch zusätzlich eine als Lösungsmittel wirkende niedermolekulare organische Komponente enthält.

Das Polymerisationsverfahren selbst spielt für die erfindungsgemäße Reinigung von Polyethern, Polyestern und/oder Polyetherestern keine besondere Rolle. Die Polymerisation kann kontinuierlich oder diskontinuierlich durchgeführt werden, und das Polymerisationssystem kann einphasig, zweiphasig oder heterogen sein.

In der Regel enthält die bei der Polymerisation gebildete Polymerlösung noch einen beträchtlichen Anteil an Monomer(en), einen großen Teil der anfangs zugesetzten Kettenabbruchsreagenz(ien) sowie gelöste Heteropolyverbindung(en). Da es vorteilhaft sein kann, die Polymerisation in Gegenwart eines Lösungsmittels durchzuführen, kann das Polymerisationssystem gegebenenfalls auch ein solches enthalten.

In einem bevorzugten Polymerisationsverfahren mit HPA-Katalyse wird so gearbeitet, daß ein Polymerisationsgemisch mit zwei flüssigen Phasen entsteht, wobei die eine die Katalysatorphase und die andere die Monomer/Polymer-Phase ist. Eine typische Monomer-/Polymer-Phase, wie sie beispielsweise bei der Ringöffnungspolymerisation von THF zu Polytetrahydrofuran (PolyTHF) in Gegenwart von Wasser mit H₃PW₁₂O₄₀ als Katalysator in einem zweiphasigen Polymerisationssystem entsteht, ist wie folgt zusammengesetzt (Angaben in Gew.-%): 77,3% THF, 21,2% PolyTHF, 1,2% H₃PW₁₂O₄₀, 0,3% Wasser.

Die Zusammensetzung der Polymerphase, die für das erfindungsgemäße Verfahren verwendet wird, hängt demgemäß von der Art des Katalysators, des oder der Monomeren, des oder der Kettenabbruchsreagenz(ien) und dem Gehalt von möglicherweise verwendetem Lösungsmittel ab und ist für das Verfahren nicht kritisch.

Nach dem erfindungsgemäßen Verfahren wird zur Reinigung der HPA-haltigen Polymerphase ein Ether zugegeben, der von so geringer Polarität ist, daß seine Zugabe zur mit HPA verunreinigten Polymerphase zum Ausfallen von HPA aus dieser Phase führt. Bevorzugt werden Ether, deren Dipolmoment kleiner als 1,4 D ist, verwendet.

Die weitere Beschaffenheit des Ethers ist beliebig. So kann beispielsweise ein einfacher Ether gleiche oder verschiedene kohlenstoffhaltige Reste aufweisen, wobei die Reste auch zu einem Ring miteinander verbunden sein können. Es kann sich bei den kohlenstoffhaltigen Resten um Alkyl-, Cycloalkyl-, Alkenyl-, Cycloalkenyl-, Aryl-, Aralkylgruppen, die gegebenenfalls durch Halogen (F, Cl, Br, I) oder durch eine oder mehrere Heteroatome (N, O, S) enthaltende Gruppen substituiert sein können, handeln. Als Beispiele seien Diethylether, Methyl-tert.-butylether, Di-tert.-butylether, Dibutylether, Dioctylether, Dicyclohexylether, Dibenzylether oder Anisol aufgeführt.

Durch Wahl von geeigneten kohlenstoffhaltigen Resten kann der zur HPA-Ausfällung dienende Ether optimal auf den jeweiligen zu reinigenden Polyether, Polyester und/oder Polyetherester abgestimmt werden.

Zur Vereinfachung des erfindungsgemäßen Verfahrens werden bevorzugt Ether eingesetzt, die bei Raumtemperatur flüssig sowie unter den Reaktionsbedingungen beständig und leicht handhabbar sind.

Die Gewichtsmenge des einzusetzenden Ethers hängt vom HPA-Gehalt und dem Gehalt anderer niedermolekularer Verbindungen, wie Monomer(en), Kettenabbruchsreagenz(ien), Lösungsmittel usw. in der zu reinigenden Polymerphase ab, beträgt aber normalerweise mindestens so viel, häufig bis doppelt so viel wie die Gesamtgewichtsmenge der anderen niedermolekularen Verbindungen. Bezogen auf die Polyether-, Polyester- und/oder Polyetherestermenge sollte die Menge an Heteroelementverbindung im allgemeinen 50 Gew.-% oder mehr, bevorzugt 100 Gew.-% oder mehr, betragen.

Liegen große Mengen niedermolekulare Verbindungen zusammen mit dem zu reinigenden Polymer vor, müßten zum Ausfällen der HPA ebenfalls große Mengen an Ether zugegeben werden. Es kann deshalb günstig sein, eine Polymer-haltige Mischung vor Zugabe des zur Ausfällung verwendeten Ethers bis zu einem Polymeranteil von mindestens 10 oder bevorzugter mindestens 50 Gew.-% und mehr zu konzentrieren.

Bei der erfindungsgemäßen Zugabe des Ethers fällt der größte Teil der in der Polymerphase gelösten HPA aus. Dabei kann es vorteilhaft sein, die Polymerphase auf geeignete Weise, beispielsweise durch herkömmliches Rühren, zu durchmischen. Für die HPA-Entfernung werden Temperaturen und Drücke gewählt, bei denen sich das Verfahren einfach durchführen läßt. Die verwendeten Temperaturen sollten nicht zu hoch sein, da die Löslichkeit der HPA in der Polymerphase mit zunehmender Temperatur steigt; normalerweise werden Temperaturen zwischen 25 und 60 °C gewählt. Die Mischung kann zur Vervollständigung der Fällung 0,1 bis 200 Stunden stehen bleiben, wobei üblicherweise 0,5 Stunden ausreichen. Häufig wird die Bildung von Emulsionen beobachtet. In diesem Fall kann die Phasentrennung durch geeignete Maßnahmen (wie Einsatz von Koaleszierfiltern) beschleunigt werden.

Durch das erfindungsgemäße Verfahren gelingt die Abtrennung des größten Teils der in der Polyether-, Polyester- und/oder Polyetherester-Phase gelösten HPA. Die HPA fällt meistens in flüssiger Phase an, die direkt in die Polymerisationsstufe zurückgeführt werden kann. Durch die Anwesenheit geringer Mengen des zur Ausfällung verwendeten Ethers in der abgesonderten HPA-Phase wird diese stabilisiert.

Wird beispielsweise die oben erwähnte, bei der THF-Polymerisation in Gegenwart von Wasser und H₃PW₁₂O₄₀ als Katalysator erhaltene THF/PolyTHF-Phase mit der doppelten Gewichtsmenge an Dioctylether versetzt, scheidet sich der größte Teil der gelösten HPA in Form einer flüssigen Phase ab. Der HPA-Restgehalt in der THF/PolyTHF/Dioctylether-Phase sinkt dabei auf 5 ppm.

Die bei der HPA-Ausfällung durch die Zugabe von Ether ablaufenden Vorgänge sind noch nicht in allen Einzelheiten aufgeklärt. Eine mögliche Wirkungsweise könnte sein, daß die Zugabe eines Ethers mit relativ geringer Polarität die Löslichkeit von Heteropolyverbindungen in der Polymerphase durch Verringerung der Polarität derselben stark herabsetzt, so daß diese aus der Polymerphase ausfallen.

Unter Heteropolysäuren im Sinne der vorliegenden Erfindung werden anorganische Polysäuren mit mindestens zwei verschiedenen Zentralatomen verstanden, welche aus schwachen, mehrbasigen Sauerstoffsäuren eines Metalls, vorzugsweise aus denen des Chroms, Molybdäns, Vanadiums und Wolframs, und/oder den entsprechenden Oxiden dieser Metalle, beispielsweise CrO₃, MoO₃, V₂O₅ oder WO₃, und denen eines anderen Metalls oder Nichtmetalls, beispielsweise Arsen, Bor, Iod, Phosphor, Selen, Silicium, Germanium oder Tellur, als gemischte, partielle Anhydride entstehen. In der Regel hat das Atomverhältnis zwischen den erstgenannten und den letztgenannten Elementen in diesen Heteropolysäuren den Wert 2,5 bis 12, vorzugsweise beträgt das Atomverhältnis 9 oder 12.

Als Beispiele für Heteropolysäuren, wie sie im erfindungsgemäßen Verfahren entfernt werden können, seien die folgenden Verbindungen genannt:
Dodecamolybdatophosphorsäure (H₃PMo₁₂O₄₀ x nH₂O),
Dodecamolybdatokieselsäure (H₄SiMo₁₂O₄₀ x nH₂O),
Dodecamolybdatocer(IV)säure (H₈CeMo₁₂O₄₂ x nH₂O),
Dodecamolybdatoarsen(V)säure (H₃AsMo₁₂O₄₀ x nH₂O),
Hexamolybdatochrom(III)säure (H₃CrMo₆O₂₄H₆ x nH₂O),
Hexamolybdatonickel(II)säure (H₄NiMo₆O₂₄H₆ x 5H₂O),
Hexamolybdatoiodsäure (H₅IMo₆O₂₄ x nH₂O),
Octadecamolybdatodiphosphorsäure (H₆P₂Mo₁₈O₆₂ x 11H₂O),
Octadecamolybdatodiarsen(V)säure (H₆As₂Mo₁₈O₆₂ x 25H₂O),
Nonamolybdatomangan(IV)säure (H₆MnMo₉O₃₂ x nH₂O),
Undecamolybdatovanadatophosphorsäure (H₄PMo₁₁VO₄₀ x nH₂O),
Decamolybdatodivanadatophosphorsäure (H₅PMo₁₀V₂O₄₀ x nH₂O),
Dodecavanadatophosphorsäure (H₇PV₁₂O₃₆ x nH₂O),
Dodecawolframatokieselsäure (H₄SiW₁₂O₄₀ x 7H₂O),
Dodecawolframatophosphorsäure (H₃PW₁₂O₄₀ x nH₂O),
Dodecawolframatoborsäure (H₅BW₁₂O₄₀ x nH₂O),
Octadecawolframatodiphosphorsäure (H₆P₂W₁₈O₆₂ x 14H₂O),
Octadecawolframatodiarsen(V)säure (H₆As₂W₁₈O₆₂ x 14H₂O),
Hexamolybdatohexawolframatophosphorsäure (H₃PMo₆W₆O₄₀ x nH₂O).

Selbstverständlich können auch Mischungen von Heteropolysäuren entfernt werden. Häufig dient das erfindungsgemäße Verfahren zur Entfernung von Dodecawolframatophosphorsäure, Dodecawolframatokieselsäure und/oder Dodecamolybdatokieselsäure, da diese aufgrund ihrer guten Verfügbarkeit bevorzugt als Katalysatoren eingesetzt werden.

Das erfindungsgemäße Verfahren wird besonders bevorzugt für die Reinigung von Polymeren verwendet, bei deren Herstellungsverfahren die freien Heteropolysäuren als Katalysatoren eingesetzt worden sind. Aber auch die Abtrennung von Alkali- und/oder Erdalkalimetallsalzen von Heteropolysäuren ist möglich.

Polyether, Polyester und Polyetherester mit einem wie vorstehend beschrieben verringerten HPA-Gehalt können durch Inkontaktbringen mit einem festen Adsorbens weiter gereinigt werden. Dabei können diese Polymere direkt oder in Lösung mit dem festen Adsorbens behandelt werden. Bevorzugt ist der Einsatz von gegebenenfalls noch lösungsmittelhaltigen Monomer/Polymer-Mischungen. Dabei können diese Mischungen, so wie sie bei der HPA-Ausfällung anfallen, aber auch in konzentrierterer oder verdünnter Form eingesetzt werden. Die in der Polymerphase vorteilhafterweise enthaltene Monomermenge sollte mindestens 10 Gew.-%, besser 50 Gew.-% und mehr, betragen.

Die Art des festen Adsorbens ist nicht beschränkt, solange es Heteropolyverbindungen adsorbieren kann. Bevorzugt sind Aktivkohlen, Aluminiumoxide, Oxide, Hydroxide und Carbonate von Erdalkali- und Seltenerdmetallen sowie basische Ionenaustauscher. Die Menge des Adsorbens hängt vom HPA-Gehalt ab und kann das 2- bis 5000fache, bevorzugt das 10- bis 1000fache, an gelöster HPA betragen. Im allgemeinen führt der Einsatz größerer Mengen an festem Adsorbens zu einem geringeren HPA-Restgehalt nach der Behandlung.

Die Temperatur bei diesem Reinigungsschritt ist nicht besonders beschränkt und sollte so gewählt werden, daß die zu behandelnde Lösung eine geeignete Viskosität aufweist. Wird ein Polyether mit mittlerer Molmasse 1000 in reiner Form eingesetzt, liegt die geeignete Temperatur normalerweise zwischen 20 und 150 °C, bevorzugt zwischen 30 und 100 °C.

Wenn das gereinigte Polymer nach der Behandlung mit einem festen Adsorbens noch Monomer oder Lösungsmittel enthält, können diese beispielsweise durch Destillation bei Normaldruck oder unter vermindertem Druck entfernt werden, wobei ein Polymer mit sehr geringem HPA-Gehalt erhalten werden kann, der unter 1 ppm, bezogen auf reinen Polyether, Polyester und/oder Polyetherester, liegen kann. Demgemäß können nach dem erfindungsgemäßen Verfahren Polyether, Polyester und/oder Polyetherester auf wirtschaftliche Weise in hoher Reinheit erhalten werden.

Alle Konzentrationsangaben der folgenden erläuternden Beispiele sind in Gew.-% angegeben. Die Versuche wurden alle unter Stickstoff durchgeführt. Die quantitative Analyse der Heteropolysäuren wurde mittels Röntgenfluoreszens und Atomabsorption durchgeführt.

### Beispiel 1

Es wurde eine bei der Polymerisation von Tetrahydrofuran (THF) zu Polytetrahydrofuran (PolyTHF) in Gegenwart von Wasser mit H₃PW₁₂O₄₀ als Katalysator anfallende Polymerphase eingesetzt, die die folgende Zusammensetzung aufwies: THF (77,3%), PolyTHF (21,2%), H₃PW₁₂O₄₀ (1,2%), Wasser (0,3%).

100 g dieser Mischung wurden mit 200 g Dioctylether versetzt. Nach 30 Stunden war der größte Teil der vorher gelösten H₃PW₁₂O₄₀ in Form einer flüssigen Mischung ausgefallen und konnte abgetrennt werden. Die THF/PolyTHF/Dioctylether-Phase wies noch einen Restgehalt von 5 ppm H₃PW₁₂O₄₀ auf. Anschließend wurde die organische Phase mit 20 g Aktivkohle (Merck) versetzt und 4 Stunden bei Raumtemperatur geschüttelt. Im nach Abtrennung der Aktivkohle und Einengung im Vakuum erhaltenen PolyTHF lag der H₃PW₁₂O₄₀-Gehalt unter 1 ppm.

### Beispiele 2 - 6

Für die Beispiele 2 - 6 wurden jeweils 100 g einer THF/PolyTHF/HPA-Lösung eingesetzt, deren Zusammensetzung in Tab. I angegeben ist. Zu dieser Mischung wurde der Ether ("Reinigungsverbindung") gegeben, und nach 50 Stunden wurde der HPA-Restgehalt in der Mischung ermittelt. Alle Proben wurden anschließend mit 20 g Aktivkohle versetzt, 4 Stunden bei Raumtemperatur geschüttelt und bei 10 mbar/140°C eingeengt. Die Analyse des so erhaltenen PolyTHF ergab einen HPA-Restgehalt von weniger als 1 ppm.

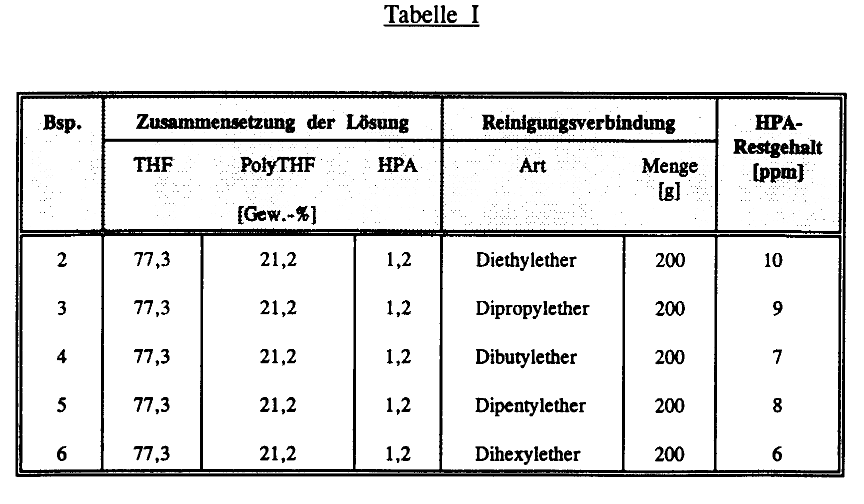

## Patentansprüche

1. Verfahren zur Entfernung von Heteropolyverbindungen aus mit Heteropolyverbindungen verunreinigten Polyethern, Polyestern und/oder Polyetherestern, dadurch gekennzeichnet, daß man die Polymere oder eine Lösung derselben mit einem Ether versetzt, der von so geringer Polarität ist, daß seine Zugabe zur Abscheidung der Heteropolyverbindung in einer gesonderten Phase führt, und die ausgefallene Heteropolyverbindungsphase abtrennt.

2. Verfahren nach Anspruch 1, dadurch gekennzeichnet, daß ein Ether der Formel R₁―O―R₂ eingesetzt wird, wobei R₁ und R₂ gegebenenfalls substituierte Alkyl-, Cycloalkyl-, Alkenyl-, Cycloalkenyl-, Aryl- und Aralkyl-Gruppen sein können, die gleich oder verschieden sind und miteinander zu einem cyclischen Rest verbunden sein können.

3. Verfahren nach Anspruch 1 oder 2, dadurch gekennzeichnet, daß der Ether ein Dialkylether, vorzugsweise ein verzweigtkettiger Dialkylether ist.

4. Verfahren nach einem der Ansprüche 1 bis 3, dadurch gekennzeichnet, daß man mit einer Ether-Gewichtsmenge versetzt, die mindestens so groß wie die Gewichtsmenge anwesender anderer niedermolekularer Verbindungen ist oder mindestens 50% der Gewichtsmenge an anwesendem Polymer beträgt.

5. Verfahren nach einem der Ansprüche 1 bis 4, dadurch gekennzeichnet, daß eine Lösung der Polymere auf einen Polymergehalt von zumindest 10 Gew.-%, bevorzugt zumindest 50 Gew.-%, eingeengt wird, bevor mit dem Ether versetzt wird.

6. Verfahren nach einem der Ansprüche 1 bis 5, dadurch gekennzeichnet, daß man das Polymer nach Abtrennung der Heteropolyverbindungsphase mit einem festen Adsorbens, das in der Lage ist, Heteropolyverbindungen zu adsorbieren, in Kontakt bringt.

7. Verfahren nach einem der Ansprüche 1 bis 6, dadurch gekennzeichnet, daß man als festes Adsorbens eines oder eine Mischung mehrerer aus den Gruppen Aktivkohlen, Aluminiumoxide, Erdalkalimetall- und Seltenerdmetalloxide, -hydroxide und -carbonate und basische Ionentauscher einsetzt.

8. Verfahren nach einem der Ansprüche 1 bis 7, dadurch gekennzeichnet, daß man nach der Entfernung der Heteropolyverbindungen das Polymer durch Abtrennen von Monomeren, Kettenabbruchsreagenzien, eventuell zugesetzten Lösungsmitteln und anderen niedrig siedenden Verbindungen erhält.

9. Verfahren nach einem der Ansprüche 1 bis 8, dadurch gekennzeichnet, daß man einen Polyoxytetramethylen-Gruppen enthaltenden Polyether einsetzt.

10. Verfahren nach einem der Ansprüche 1 bis 9, dadurch gekennzeichnet, daß die abgetrennte Heteropolyverbindungsphase als Polymerisationskatalysator wiederverwendet wird.

## Claims

1. A method of removing heteropoly compounds from polyethers, polyesters and/or polyether esters which are contaminated with heteropoly compounds, which comprises adding, to the polymers or a solution thereof, an ether whose polarity is so low that its addition leads to the deposition of the heteropoly compound in a separate phase, and separating off the precipitated heteropoly compound phase.

2. A method as claimed in claim 1, wherein an ether of the formula R₁-O-R₂ is employed, in which R₁ and R₂ are unsubstituted or substituted alkyl, cycloalkyl, alkenyl, cycloalkenyl, aryl or aralkyl which are identical or different and may be linked to one another to form a cyclic radical.

3. A method as claimed in claim 1 or 2, wherein the ether is a dialkyl ether, preferably a branched-chain dialkyl ether.

4. A method as claimed in any of claims 1 to 3, wherein the amount by weight of ether added is at least as large as the amount by weight of other low molecular mass compounds present or is at least 50% of the amount by weight of polymer present.

5. A method as claimed in any of claims 1 to 4, wherein a solution of the polymers is concentrated to a polymer content of at least 10% by weight, preferably at least 50% by weight, before the ether is added.

6. A method as claimed in any of claims 1 to 5, wherein after the heteropoly compound phase has been separated off the polymer is brought into contact with a solid adsorbent capable of adsorbing heteropoly compounds.

7. A method as claimed in any of claims 1 to 6, wherein a solid adsorbent or a mixture of two or more solid adsorbents from the groups consisting of activated carbons, aluminas, alkaline earth metal and rare earth metal oxides, hydroxides and carbonates, and basic ion exchangers is employed.

8. A method as claimed in any of claims 1 to 7, wherein after the heteropoly compounds have been removed the polymer is obtained by separation from monomers, chain termination reagents, any solvents added, and other low-boiling compounds.

9. A method as claimed in any of claims 1 to 8, wherein a polyether is employed which includes polyoxytetramethylene groups.

10. A method as claimed in any of claims 1 to 9, wherein the separated heteropoly compound phase is reused as polymerization catalyst.

## Revendications

1. Procédé d'élimination d'hétéropolycomposés à partir de polyéthers, polyesters et/ou polyétheresters pollués par des hétéropolycomposés, caractérisé en ce que l'on ajoute aux polymères ou à une solution de ceux-ci, un éther d'une polarité si faible que son ajout mène à la séparation de l'hétéropolycomposé en une phase distincte, puis on sépare la phase de l'hétéropolycomposé ayant précipité.

2. Procédé selon la revendication 1, caractérisé en ce que l'on ajoute un éther de formule R₁-O-R₂, où R₁ et R₂ peuvent représenter des groupements alkyle, cycloalkyle, alcényle, cycloalcényle, aryle et aralkyle éventuellement substitués, identiques ou différents, et peuvent être liés ensemble en un reste cyclique.

3. Procédé selon la revendication 1 ou 2, caractérisé en ce que l'éther est un dialkyléther, de préférence un dialkyléther à chaîne ramifiée.

4. Procédé selon l'une quelconque des revendications 1 à 3, caractérisé en ce que l'on ajoute une quantité en poids d'éther qui est au moins aussi importante que la quantité en poids des autres composés à bas poids moléculaire présents, ou qui représente au moins 50% de la quantité en poids des polymères présents.

5. Procédé selon l'une quelconque des revendications 1 à 4, caractérisé en ce que l'on concentre une solution de polymères jusqu'à une teneur en polymères d'au moins 10% en poids, de préférence d'au moins 50% en poids, avant ajout de l'éther.

6. Procédé selon l'une quelconque des revendications 1 à 5, caractérisé en ce que l'on met en contact le polymère, après séparation de la phase de l'hétéropolycomposé, avec un adsorbant solide susceptible d'adsorber les hétéropolycomposés.

7. Procédé selon l'une quelconque des revendications 1 à 6, caractérisé en ce que l'on utilise en tant qu'adsorbant solide l'un ou un mélange de plusieurs composés choisis dans les groupes formés par les charbons actifs, les oxydes d'aluminium, les oxydes, hydroxydes et carbonates de métaux alcalino-terreux et de terres rares et les échangeurs d'ions basiques.

8. Procédé selon l'une quelconque des revendications 1 à 7, caractérisé en ce que l'on obtient le polymère, après élimination des hétéropolycomposés, par séparation des monomères, des réactifs d'interruption de chaîne, des éventuels solvants ajoutés et d'autres composés à bas point d'ébullition.

9. Procédé selon l'une quelconque des revendications 1 à 8, caractérisé en ce que l'on utilise un polyéther contenant des groupements polyoxytétraméthylène.

10. Procédé selon l'une quelconque des revendications 1 à 9, caractérisé en ce que la phase de l'hétéropolycomposé séparé est réutilisée en tant que catalyseur de polymérisation.
